# EUROPEAN PATENT APPLICATION

(11) **EP 2 698 183 A2**
(43) Date of publication of application: **19.02.2014**
(21) Application number: 12771780.9
(22) Date of filing: 13.04.2012
(51) Int. Cl.: A61N 7/00, A61B 18/00, A61B 19/00

(54) **APPARATUS FOR ULTRASONIC THERAPEUTIC TREATMENT, AND METHOD FOR DRIVING THE APPARATUS**

(30) Priority: 15.04.2011 KR 20110035305
(71) Applicant: Alpinion Medical Systems Co., Ltd., Hwaseong-si, Gyeonggi-do 445-380 (KR)
(72) Inventor: JUN, Sukhwan, Incheon 402-200 (KR)
(74) Representative: Thorniley, Peter
(86) International application number: PCT/KR2012/002799
(87) International publication number: WO 2012/141518

(57) **Abstract**

The present disclosure in some embodiments relates to an ultrasonic medical device and to a method of operating the same. The medical device comprises a scanline information processor, comparison unit and treatment parameter setup unit. The scanline information processor acquires z-axis data from diagnosis information of a patient and processes information related to the z-axis data which represents a depth of a treatment position on a scanline image selected for treating the patient. The comparison unit makes a comparison of the z-axis data acquired in real time with positional data of an initial treatment position in a therapy site under treatment for the patient and determines whether z-axis variations deviate from a predetermined range. The treatment parameter setup unit provides setup information for adjusting the level of treatment for the patient, based on the z-axis variations.

## Description

### [Technical Field]

The present disclosure in some embodiments relates to an ultrasonic medical device and to a method for operating the same. More particularly, the present disclosure relates to an ultrasonic medical device capable of performing an effective targeted treatment of a lesion through a real-time monitoring of the therapy site in a high-intensity focused ultrasound (HIFU) treatment, for example, and to a method for operating the ultrasonic medical device.

### [Background]

The statements in this section merely provide background information related to the present disclosure and may not constitute prior art.

An ultrasound diagnosis and treatment on the human body are under active research and development since it obviates the need for an incision of a human body and thus leaves neither a surgical scar nor the concern for secondary infections in general. Exemplary applications of the ultrasonic wave or ultrasound for the medical field are a diagnosis area such as a fetal diagnosis or a cancer diagnosis and a treatment area such as a lipectomy or destruction of a cancer tissue or malignant tumor.

A HIFU (High-Intensity Focused Ultrasound) is a surgical treatment of burning cancer cells by intensively irradiating the ultrasonic wave on the cancer tissue. Although the HIFU has originally been developed for treating a prostate cancer, it has been gradually extending its applications to a non-solid tumor such as a brain cancer, a uterine myoma and an arrhythmia beyond a solid cancer such as a liver cancer, a breast cancer and a pancreatic cancer and the like. Especially, the HIFU treatment now presents an excellent result in the treatment of a liver cancer and a pancreatic cancer for which a surgical operation is scarcely applicable.

The HIFU treatment has various aspects such as a thermal effect, a cavitation effect, a mechanical effect, a capillary destruction nearby a tumor and an immunity effect. Herein, the thermal effect includes the coagulation of a blood vessel and the necrosis of tumor cells by use of heat above 65 degrees. The cavitation effect is associated with pressurizing cells to cause cell protein to be denatured and thereby destroying DNAs of the tumor. In addition, the mechanical effect is to break the chemical link between cancer cells. The capillary destruction adjacent to the tumor is to prevent the tumor from proliferating by destroying the adjacent capillary so as not to supply nutrition to the tumor as well as a lesion to be treated. The immunity effect relates to increasing the level of immunity as with increasing lymphocyte by recognizing the tumor cell destroyed after the treatment as an antigen. Among these effects, the thermal effect is one of the most popular kinds.

Furthermore, the state of the art HIFU treatment so far applies motion tracking technology for performing treatment by detecting the respiration or movement of the patient. However, current HIFU treatments lack consideration for the respirations of the internal organs or involuntary movements due to physiological nature of the body, which practically limits an intensive treatment on particular lesions. Then, the involuntary movements left unattended during the HIFU treatment end up damaging the surrounding normal tissue.

### [Disclosure]

### [Technical Problem]

The embodiments of the present disclosure provide an ultrasonic medical device capable of effectively carrying out a targeted HIFU treatment of a specific lesion by monitoring the movement of a therapy site in real time using a selected scanline image and is capable of stopping the ultrasound therapy by sensing excessive site movements with real-time ultrasonic scanline images, and a method for driving the device.

### [Summary]

According to an aspect of the present disclosure, there is provided an ultrasonic medical device comprising a scanline information processor, comparison unit and treatment parameter setup unit. The scanline information processor acquires z-axis data from diagnosis information of a patient and processes information related to the z-axis data which represents a depth of a treatment position on a scanline image selected for treating the patient. The comparison unit makes a comparison of the z-axis data acquired in real time with positional data of an initial treatment position in a therapy site under treatment for the patient and determines whether z-axis variations deviate from a predetermined range. The treatment parameter setup unit provides setup information for adjusting the level of treatment for the patient, based on the z-axis variations.

Further, according to another aspect of the present disclosure, there is provided a method for operating an ultrasonic medical device, comprising acquiring z-axis data from diagnosis information of a patient and processing information related to the z-axis data which represents a depth of a treatment position on a scanline image selected for treating the patient. The method further comprises making a comparison of the z-axis data acquired in real time with a positional data of an initial treatment position in a therapy site under treatment for the patient and making a determination of whether z-axis variations deviate from a predetermined range. The method further comprises providing setup information for adjusting a level of the treatment for the patient, based on the z-axis variations.

### [Advantageous Effects]

According to some embodiments of the present disclosure, a HIFU treatment can be performed by taking account of involuntary movements of the internal organs of the body to thereby minimize damages of nearby normal tissues of the target lesion.

Moreover, the present disclosure can stop the HIFU treatment by detecting patient's excessive movements associated with e.g. an electric steering region through real-time ultrasonic scanline images and thereby maximizes the treatment performance.

### [Description of Drawings]

FIG. 1 is an exemplary diagram of an ultrasonic medical device according to at least one embodiment.
FIG. 2 is a block diagram of a configuration of a controller body of FIG. 1.
FIG. 3 is a flowchart of procedures of the operation of the medical device of FIG. 1.
FIG. 4 is a view of an electric steering region.
FIG. 5 is a view of comparing ultrasonic images before and after taking account of involuntary movements of the internal organs of the body.

### [Detailed Description]

Hereinafter, some embodiments of the present disclosure will be described in detail with reference to the accompanying drawings. In the following description, like reference numerals designate like elements although the elements are shown in different drawings. Further, in the following description of the embodiments, a detailed description of known functions and configurations incorporated herein will be omitted for the purpose of clarity and for brevity.

Additionally, in describing the components of the present disclosure, terms like first, second, A, B, (a), and (b) are used. These are solely for the purpose of differentiating one component from another, and one of ordinary skill would understand the terms are not to imply or suggest the substances, order or sequence of the components. If a component is described as 'connected', 'coupled', or 'linked' to another component, one of ordinary skill in the art would understand the components are not necessarily directly 'connected', 'coupled', or 'linked' but also are indirectly 'connected', 'coupled', or 'linked' via a third component.

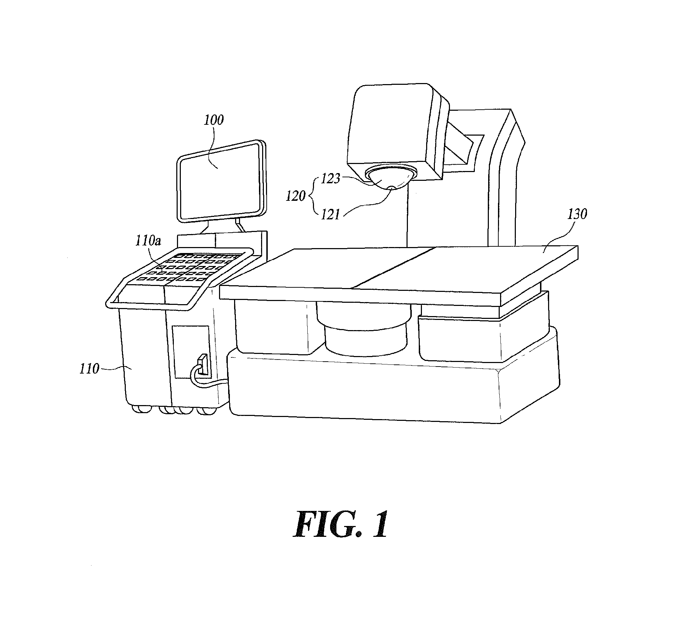

FIG. 1 is an exemplary diagram of an ultrasonic medical device according to at least one embodiment.

Referring to FIG. 1, the ultrasonic medical device according to one or more embodiments of the present disclosure includes a display unit 100, a controller body 110 and a treatment unit 120, and may further include a treatment table 130 on which a patient is placed for treatment or diagnosis and a measuring device (not shown) for obtaining diagnosis information of the patient.

Herein, the display unit 100 is a display device like a computer monitor. The display unit 100 may display patient's diagnosis information provided upon acquiring from a monitoring unit 121. The display unit 100 further displays patient's additional information inputted to a patient monitoring module (not shown) by a hospital medical team or the operations staff or the patient diagnosis information provided on-line over a connection with e.g., a server of another medical institution. The display unit 100 can also display the result of the patient about the therapy site under treatment.

A controller body 110 may include a key input unit 110a for manipulating diagnosis or treatment. It controls and manages to process information on a patient such as diagnosis information, in particular, information on involuntary movements of the internal organs of the body monitored in real time through the monitoring unit 121 and to display the same information on the display unit 100. In addition, the controller body 110 performs adjusting the level of, for example, an ultrasonic transmission through a therapeutic wave transmission unit 123 by analyzing the diagnosis information of the patient acquired in real time. "Adjusting" as used herein means adjusting the focus position of the ultrasonic irradiation in real time by taking account of involuntary movements of the internal organs of the body.

The treatment unit 120 includes a therapeutic wave transmission unit 123 and may further include a monitoring unit 121. Herein, the monitoring unit 121 including e.g., a CCD (Charge Coupled Device) camera acquires a reflected wave of a specific beam or a diagnosis pulse after it is transmitted on a specific part or area of the patient body. The acquired information may be supplied to the controller body 110. In this way, the monitoring unit 121 monitors in real time the state of diagnosis of the patient placed on the treatment table 130. In addition, for example, the therapeutic wave transmission unit 123 includes more than e.g., 512 channels of array elements configured to transmit ultrasonic waves or ultrasounds, and thereby high power multi-focal outputs are converged under the control of the controller body 110 and then transmitted to the therapy site of the patient body in high intensity with the focusing position of the ultrasonic transmission being controlled by new parameter values set up by taking into account of the involuntary movements of the internal organs.

Besides the aforementioned components, the ultrasonic medical device according to at least one embodiment may include a separate diagnostic device such as a probe or transducer for acquiring patient diagnosis information.

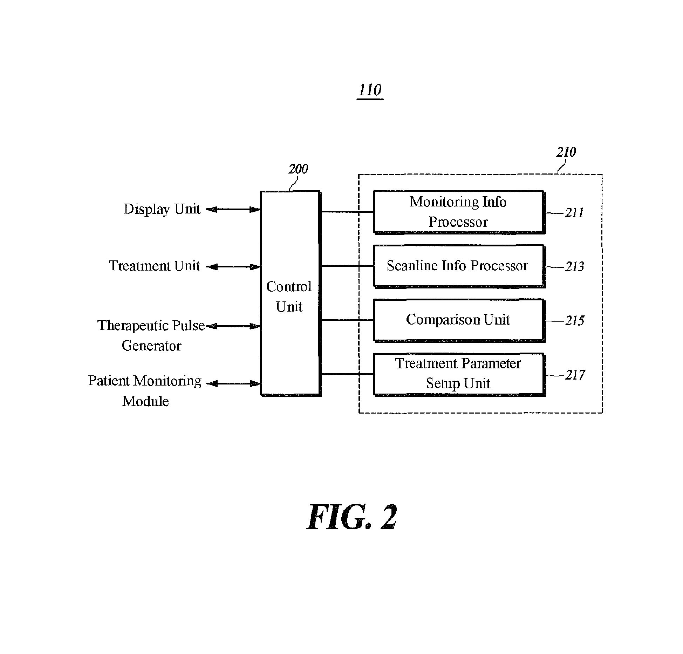

FIG. 2 is a block diagram of a configuration of a controller body of FIG. 1.

Referring to FIG. 2 together with FIG. 1, the controller body 110 according to one or more embodiments of the present disclosure includes a control unit 200 and an involuntary movement information processing unit 210 and further includes a therapeutic pulse generator (not shown) and a patient monitoring module (not shown).

The control unit 200 controls signals or information processed in the controller body 110. For example, the control unit 200 has a specific area of the patient's body monitored and renders the information on the involuntary movements to be displayed on the display unit 100 and thereby generates the therapeutic pulses at different focusing positions depending on the analyzed involuntary movement information in order to treat the patient. For example, the control unit 200 commands the therapeutic pulse generator to generate therapeutic pulses with different focusing positions and supply the generated pulses to the therapeutic wave transmission unit 123 according to the setup information provided by a treatment parameter setup unit 217 included in the involuntary movement information processing unit 210.

The involuntary movement information processing unit 210 further includes a monitoring information processor 211, a scanline information processor 213, a comparison unit 215 and the treatment parameter setup unit 217.

Herein, the monitoring information processor 211 processes the patient diagnosis information acquired and provided by the monitoring unit 121. In addition, diagnosis-related information may be provided in the form of, e.g., a high resolution 3D image, to the display unit 100 under the control of the control unit 200. Further, the monitoring information processor 211 processes the patient diagnosis information supplied through an additional measuring device as well as patient diagnosis information supplied from a patient monitoring module before supplying the information to the display unit 100. Herein, the patient monitoring module stores and manages the patient diagnosis information which is supplied through separate input processes to the hospital medical team or the operations staff or provided on-line over a connection with e.g., a server of another medical institution.

For example, the scanline information processor 213 acquires and processes information on a scanline at the lesion location by using the patient diagnosis information provided by the monitoring information processor 211. More specifically, value along the z-axis or depth of the location to be treated is acquired and processed from a selected scanline image.

The comparison unit 215 performs the processes of receiving from the scanline information processor 213 in real time the z-axis data from the selected scanline image and comparing the z-axis data with the initial position of the lesion. For example, the initial lesion position prior to an involuntary movement of a particular organ, which is set to A, is compared with regard to depth with altered position B defined by taking account of the involuntary movement to determine whether the altered position B is deeper or shallower than the initial position A. Further, the comparison unit 215 determines whether z-axis variations deviate from a range of an electric steering region. The electric steering range of such region can be set up and stored in the initial system design.

The treatment parameter setup unit 217 sets up information for use in controlling the treatment level or intensity of the patient, based on the z-axis variations. For example, information on changing parameters can be set corresponding to the z-axis variations in real time. For example, when the scanline information processor 213 or the comparison unit 215 provides the information on the z-axis variations, the treatment parameter setup unit 217 provides setup information corresponding to the relevant z-axis variation to the control unit 200 which then performs treatment of the patient by generating therapeutic pulses with different depths depending on the setup information. To this end, the treatment parameter setup unit 217 may store and manage the setup information in the form of a lookup table.

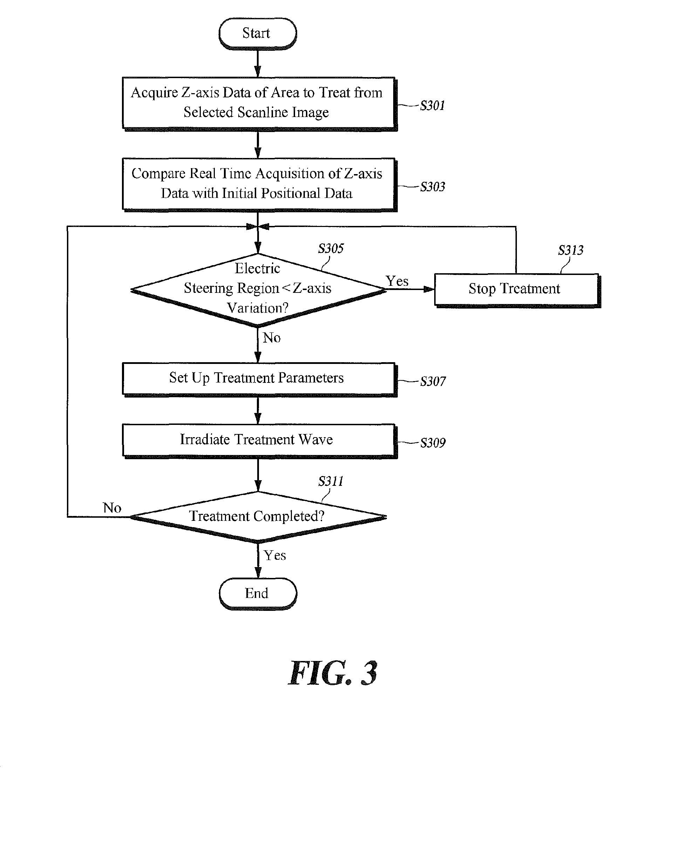

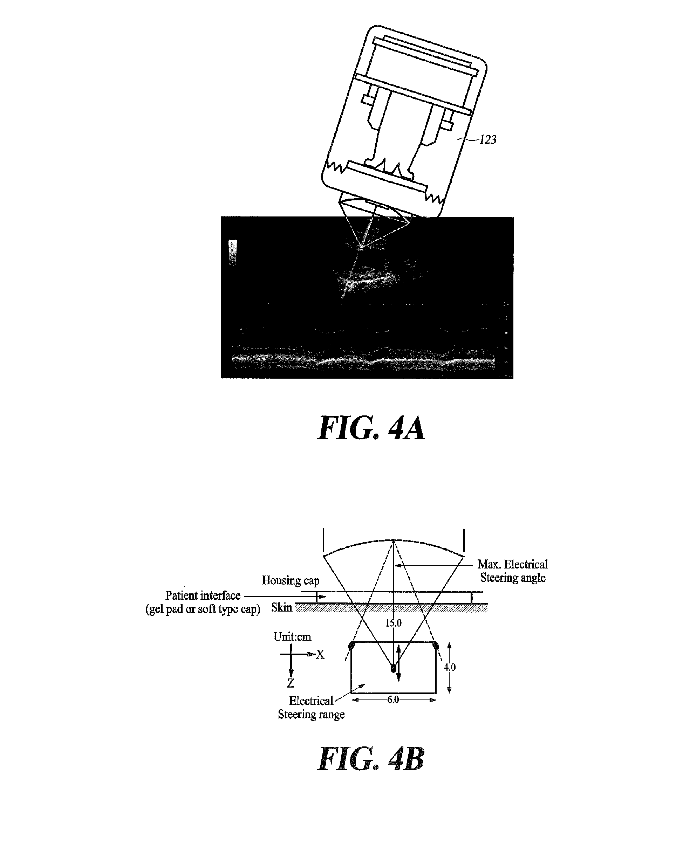

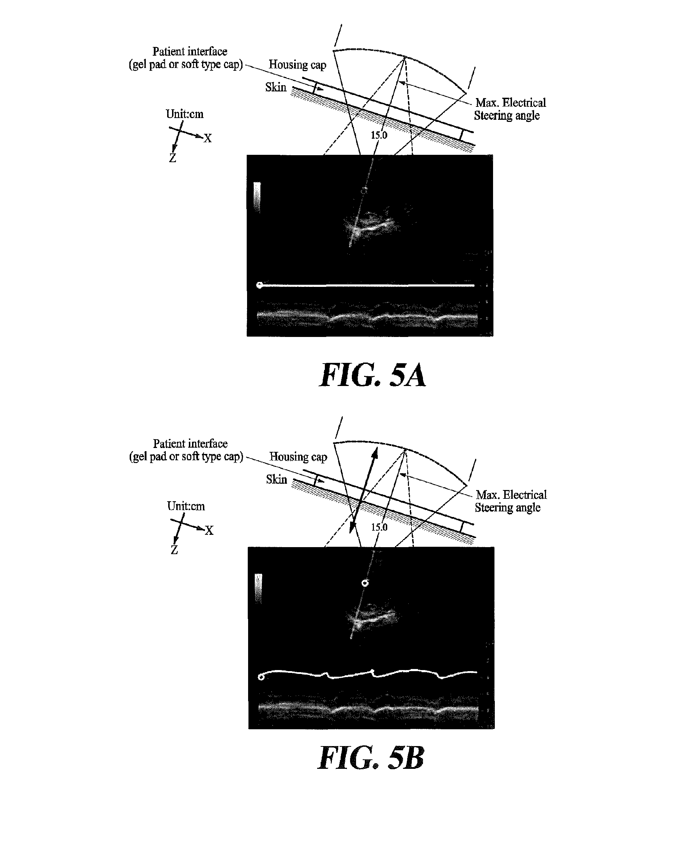

FIG. 3 is a flowchart of procedures of the operation of the medical device of FIG. 1; FIG. 4 is a view of an electric steering region and FIG. 5 is a view of comparing ultrasonic images before and after taking account of involuntary movements of the internal organs of the body.

Referring to FIGs. 3 to 5 together with FIGs. 1 and 2, the medical device may start with a sonication stand-by stage for the HIFU treatment by monitoring the diagnosis information of a patient placed on a treatment table 130. Alternatively, the medical device may cooperate with the patient monitoring module to receive the patient diagnosis information for the purpose of treating the patient on the treatment table 130.

Then, the medical device acquires from the diagnosis information z-axis data of the treatment position on the selected scanline image at the treatment position in real time (in step S301).

Thereafter, the medical device compares the z-axis data acquired in real time taking account of involuntary movements with the initial positional data of lesion prior to the involuntary movement and thereby tracks real-time positional shifts of a particular organ such as a liver (S303).

In this process of tracking the positional shifts or movements, the medical device determines whether the z-axis variation deviates from the range of the electric steering region (S305).

Referring to FIG. 4 in this context, (a) illustrates the electric steering region in relation to a B-mode image and (b) illustrates the electric steering region in relation to the therapeutic wave transmission unit 123 where the electric steering region is meant to include the lateral as well as depthwise regions. In FIG. 4(b), the x-axis represents time axis and the z-axis represents the depth of the subject body. For example, a deviation from the electric steering region will harm the normal tissue and, in response, the medical device according to at least one embodiment of the present disclosure additionally determines whether there is a deviation from the electric steering region so as to prevent any damages to the normal tissue.

When the z-axis variation is determined not to deviate from the range of the electric steering region, the medical device can set up the parameter value depending on the real-time z-axis variation (S307). Herein, the parameter setup may mean extracting and outputting parameter values corresponding to z-axis variations in real time.

With the set parameter value, the medical device controls the treatment wave depending on the parameter values corresponding to the z-axis variations acquired in real time and transmits the controlled treatment wave to the lesion of the patient (S309).

When this process completes the treatment (S311), the medical device terminates the operation.

However, if the z-axis variation deviates from the electric steering range in step S305, the medical device stops the treatment (S313) and thereby prevents any damages to the normal tissue neighboring the lesion.

Referring to FIG. 5 where encircled areas represent the initial position of a lesion, (a) illustrates a result of the treatment performed regardless of involuntary movements of the internal organ, causing damages to the nearby tissue of the lesion, whereas (b) illustrates the positional shifts of the lesion being correctly imaged and tracked with time by taking account of the involuntary movements of the internal organ.

In this way, by considering the positional shifts of the lesion as occurred by the involuntary movements, the intensity or depth of the treatment wave for treating the lesion can be controlled to complete the treatment to prevent damages at nearby normal tissue besides the lesion, and this process additionally takes account of the electric steering region in the lateral as well as depthwise directions of the lesion in order to maximize the treatment efficiency.

Although exemplary embodiments of the present disclosure have been described for illustrative purposes, those skilled in the art will appreciate that various modifications, additions and substitutions are possible, without departing from the various characteristics of the disclosure. Therefore, exemplary embodiments of the present disclosure have been described for the sake of brevity and clarity. Accordingly, one of ordinary skill would understand the scope of the disclosure is not limited by the explicitly described above embodiments but by the claims and equivalents thereof.

In addition, one of ordinary skill would understand terms like 'include', 'comprise', and 'have' to be interpreted in default as inclusive or open rather than exclusive or closed unless expressly defined to the contrary. All the terms that are technical, scientific or otherwise agree with the meanings as understood by a person skilled in the art unless defined to the contrary. One of ordinary skill would understand common terms as found in dictionaries are interpreted in the context of the related technical writings not too ideally or impractically unless the present disclosure expressly defines them so.

### Industrial Applicability

The embodiments of the present disclosure are applicable to a medical device and a method for operating the same. According to the embodiments of the present disclosure, HIFU treatment can be performed by taking account of involuntary movements of the internal organ of the body, whereby minimizing damages to normal tissue neighboring the lesion. In addition, stopping the treatment by detecting excessive bodily movements in relation to an electric steering region or others by using real-time ultrasonic scanline images can maximize the treatment effect.

### CROSS-REFERENCE TO RELATED APPLICATION

If applicable, this application claims priority under 35 U.S.C §119(a) of Patent Application No. 10-2011-0035305, filed on April 15, 2011 in Korea, the entire content of which is incorporated herein by reference. In addition, this non-provisional application claims priority in countries, other than the U.S., with the same reason based on the Korean Patent Application, the entire content of which is hereby incorporated by reference.

## Claims

1. An ultrasonic medical device, comprising:
a scanline information processor configured to acquire z-axis data from a diagnosis information of a patient and to process an information related to the z-axis data which represents a depth of a treatment position on a scanline image selected for treating the patient;
a comparison unit configured to make a comparison of the z-axis data acquired in real time with a positional data of an initial treatment position in a therapy site under treatment for the patient and to determine whether z-axis variations deviate from a predetermined range; and
a treatment parameter setup unit configured to provide a setup information for adjusting a level of the treatment for the patient, based on the z-axis variations.

2. The ultrasonic medical device of claim 1, further comprising:
a monitoring information processor configured to monitor the patient to generate the diagnosis information.

3. The ultrasonic medical device of claim 2, further comprising:
a monitoring unit including a CCD (Charge Coupled Device) camera or a probe for use in monitoring the patient.

4. The ultrasonic medical device of claim 1, wherein the initial treatment position in the therapy site is a first site of a lesion prior to an involuntary movement of an organ.

5. The ultrasonic medical device of claim 1, wherein the comparison unit performs the comparison of the z-axis data acquired in real time with the positional data of the initial treatment position in the therapy site under treatment for the patient, by setting a first site of a lesion prior to an involuntary movement of a specific organ and making a depth comparison of a second altered site defined by taking account of the involuntary movement relative to the first site.

6. The ultrasonic medical device of claim 5, wherein the comparison unit determines whether the z-axis shift deviates from the predetermined range of an electric steering region.

7. The ultrasonic medical device of claim 1, wherein the therapeutic parameter setup unit is in the form of a lookup table for storing and managing the setup information.

8. The ultrasonic medical device of claim 1, further comprising:
a therapeutic pulse generator configured to generate different therapeutic pulses according to the setup information provided by the treatment parameter setup unit; and
a therapeutic wave transmission unit configured to irradiate the different therapeutic pulses with correspondingly different focusing positions on a lesion of the patient.

9. A method for operating an ultrasonic medical device, comprising:
acquiring z-axis data from a diagnosis information of a patient and processing an information related to the z-axis data which represents a depth of a treatment position on a scanline image selected for treating the patient;
making a comparison of the z-axis data acquired in real time with a positional data of an initial treatment position in a therapy site under treatment for the patient and making a determination of whether z-axis variations deviate from a predetermined range; and
providing a setup information for adjusting a level of the treatment for the patient, based on the z-axis variations.

10. The method of claim 9, wherein the making of the comparison of the z-axis data acquired in real time with the positional data of the initial treatment position in the therapy site under treatment for the patient comprises setting a first site of a lesion prior to an involuntary movement of a specific organ and making a depth comparison of a second altered site defined by taking account of the involuntary movement relative to the first site.

11. The method of claim 9, wherein the making of the determination determines whether the z-axis shift deviates from the predetermined range of an electric steering region.

12. The method of claim 9, wherein further comprising:
generating different therapeutic pulses according to the setup information; and
irradiating the different therapeutic pulses with correspondingly different focusing positions on a lesion of the patient.

13. The method of claim 9, further comprising:
stopping the treatment of the patient when the z-axis shift deviates from the predetermined range.
